# EUROPÄISCHE PATENTSCHRIFT

(11) **EP 3 179 849 B1**
(45) Veröffentlichungstag und Bekanntmachung des Hinweises auf die Patenterteilung: **09.01.2019**
(21) Anmeldenummer: 15778195.6
(22) Anmeldetag: 11.08.2015
(51) Int. Cl.: A01K 29/00, A22B 3/00

(54) **VORRICHTUNG ZUR BETÄUBUNGSKONTROLLE EINES SCHLACHTTIERES**
DEVICE FOR CONTROLLING STUNNING OF AN SLAUGHTER ANIMAL
APPAREIL POUR LE CONTRÔLE DE L'ANESTHÉSIE D'UN ANIMAL DE BOUCHERIE

(30) Priorität: 13.08.2014 DE 202014006472 U
(43) Veröffentlichungstag der Anmeldung: 21.06.2017
(73) Patentinhaber: CSB-System AG, 52511 Geilenkirchen (DE)
(72) Erfinder: SCHIMITZEK, Peter, 52511 Geilenkirchen (DE)
(74) Vertreter: Weihrauch, Frank
(86) Internationale Anmeldenummer: PCT/DE2015/000398
(87) Internationale Veröffentlichungsnummer: WO 2016/023534

(56) Entgegenhaltungen:
- EP-A2- 2 389 809
- WO-A1-2013/052001
- WO-A1-2014/037015
- DE-A1-102011 077 944
- DE-U1-202013 002 484

## Beschreibung

Die Erfindung betrifft eine Vorrichtung zur Betäubungskontrolle eines Schlachttieres innerhalb einer Schlachttierverarbeitung.

Bei einer Schlachttierverarbeitung ist es in der Regel vorgeschrieben, die Schlachttiere vor dem eigentlichen Tötungsvorgang zu betäuben.
Die Betäubung erfolgt hierbei, je nach Art des Schlachttieres, durch Bolzenschuss, Elektrobetäubung oder mittels CO₂-Betäubung. Die Betäubung dient insbesondere dazu, unnötiges Leiden des Tieres während der Tötung zu vermeiden.

In diesem Zusammenhang liegt der vorliegenden Erfindung das Problem zugrunde, dass die durchgeführte Betäubung des Schlachttieres mitunter fehlschlagen oder nur unzureichend lange anhalten kann, sodass das Schlachttier unter Umständen, insbesondere während des Entblutens, das Bewusstsein zurückerlangt. Um dies zu verhindern ist in der Europäischen Union inzwischen vorgeschrieben, dass in den Schlachthöfen eine Betäubungskontrolle durchgeführt wird. Eine solche Betäubungskontrolle sieht unter anderem ein Überwachungsverfahren und regelmäßige Kontrollen der Betäubungsqualität vor.

Aus dem Stand der Technik ist es bekannt, dass eine derartige Betäubungskontrolle durch entsprechend geschultes Personal durchgeführt wird, welches das jeweilige Schlachttier nach der Betäubung und während des Entblutens auf eventuell auftretende Reaktionen oder Symptome überwacht.
Ergibt die Überwachung, dass die Betäubung unzureichend war, wird das Schlachttier nachbetäubt.

Eine derartige Überwachung der Betäubung ist jedoch sehr personalaufwändig und dementsprechend teuer in der Durchführung.

Zudem kann es bei einer subjektiven Beurteilung der Betäubungsqualität auch zu Fehlinterpretationen durch das zuständige Personal kommen.

In der Druckschrift WO 2014/037015 A1 wird zudem eine Vorrichtung zur Betäubungskontrolle von Schlachttieren, insbesondere von Geflügel, beschrieben. Dabei ist es vorgesehen, das Geflügel in eine gasgefüllte Betäubungszone zu transportieren, innerhalb welcher eine Messung und Anpassung der Gaskonzentration zur Gewährleistung einer optimalen Betäubung stattfindet. Zusätzlich ist eine visuelle Kontrolle der Wirksamkeit der Betäubung, insbesondere mittels einer Kamera, vorgesehen.

Die Aufgabe der vorliegenden Erfindung besteht darin, eine Vorrichtung zur Betäubungskontrolle eines Schlachttieres bereitzustellen, welche eine zuverlässige, schnelle und kostengünstige Kontrolle der Betäubung eines Schlachttieres innerhalb einer Schlachttierverarbeitung ermöglicht.

Die Aufgabe wird durch eine Vorrichtung mit den in dem Patentanspruch 1 aufgeführten Merkmalen gelöst. Bevorzugte Weiterbildungen ergeben sich aus den Unteransprüchen.

Eine erfindungsgemäße Vorrichtung zur Betäubungskontrolle eines Schlachttieres ist innerhalb einer Schlachttierverarbeitung nach einer Betäubungsstation vorgesehen, sodass eine Kontrolle der Betäubung, insbesondere vor dem Entblutungsvorgang oder vor dem Brühen, durchführbar ist.
Die Vorrichtung ist hierbei derart in den Schlachttierverarbeitungsprozess eingebunden, dass das zu überprüfende Schlachttier, auf dem Weg seines Transports, vorzugsweise entlang einer herkömmlichen Rohrbahn, den Wirkungsbereich der Vorrichtung durchläuft.

Die Vorrichtung weist erfindungsgemäß zum Ersten eine Stimulationseinheit auf, mit welcher ein Stimulationsreiz an das Schlachttier abgebbar ist.

Unter einem Stimulationsreiz wird im vorliegenden Zusammenhang ein Reiz verstanden, auf welchen das Schlachttier bei einer unzureichenden Betäubung mit entsprechenden körperlichen Reaktionen, beispielsweise einer zuckenden Bewegung, reagieren würde.

Des Weiteren weist die erfindungsgemäße Vorrichtung eine Bilderfassungseinheit mit einem Bilderfassungsbereich auf, wobei die Bilderfassungseinheit erfindungsgemäß als 3D-Kamera ausgebildet ist.
Die 3D-Kamera bietet hierbei den besonderen Vorzug, dass zum einen insbesondere die Aktivbewegung des Schlachttieres besonders fein, das bedeutet im Millimeterbereich, detektierbar ist und dass weiterhin eine Bewegungsdetektion auch ohne festgelegte Referenzpunkte durchführbar ist.
Zum anderen sind mittels einer 3D-Kamera auch Aktivbewegungen des Schlachttieres parallel zur optischen Achse der 3D-Kamera, also Bewegungen auf die Kamera zu oder von der Kamera weg, detektierbar.

Weitere Vorteile der 3D-Kamera bestehen zudem darin, dass diese bereits innerhalb des Bildes metrisch kalibrierte Daten, wie beispielsweise die Koordinaten eines Bildpunktes als x-, y- und z-Werte in einem euklidischen Koordinatensystem, bereitstellen können. Dies erleichtert in der Auswertung die Differenzierung zwischen Aktivbewegungen und transportbedingten passiven Bewegungen.

Einen weiteren Vorteil bildet, dass diese, je nach Bauform, einen integrierten Musterprojektor aufweisen können, sodass dann auf eine externe Lichtquelle zur Beleuchtung des Schlachttieres verzichtet werden kann. Auf diese Weise können zum einen die Bereitstellungskosten für eine erfindungsgemäße Vorrichtung zur Betäubungskontrolle niedrig gehalten werden. Zum anderen kann durch den Verzicht auf eine externe Lichtquelle die Vorrichtung entsprechend klein ausgebildet werden. Ebenso kann bei der vorliegenden Ausbildung der Erfindung als weiterer Vorteil auf eine Hintergrundplatte, welche im Allgemeinen eine optische Detektion des Schlachttieres als Ganzes erleichtern und Mitarbeiter vor einer möglichen Blendung durch eine Beleuchtung schützen soll, verzichtet werden.

In einer bevorzugten Weiterbildung können zwei Bilderfassungseinheiten mit jeweils zugehörigem Bilderfassungsbereich angeordnet und mit der Auswertungseinheit verbunden sein, wobei die optischen Achsen der zwei Bilderfassungseinheiten in einem Winkel, beispielsweise orthogonal zueinander angeordnet sind, um so Aktivbewegungen des Schlachttieres besonders zuverlässig detektieren zu können.

In dem Bilderfassungsbereich ist ein Abschnitt einer Oberfläche des Schlachttieres optisch erfassbar, wobei in dem Abschnitt der Oberfläche diskrete Bildpunkte, mit deren Raumkoordinaten und vorzugsweise einem Helligkeits- oder Farbwert, erfassbar sind.
Die erfassten Bildpunkte werden durch die Bilderfassungseinheit anschließend als Bildpunktdaten übertragbar bereitgestellt.

Als weiteren Bestandteil weist die erfindungsgemäße Vorrichtung eine Auswertungseinheit auf.
Die Auswertungseinheit ist mit der Bilderfassungseinheit verbunden und dazu in der Lage, zum einen die, durch die Bilderfassungseinheit bereitgestellten, Bildpunktdaten zu erfassen und zum anderen aus den erfassten Bildpunktdaten eine Aktivbewegung des Schlachttieres zu ermitteln.

Unter einer Aktivbewegung wird vorliegend eine Eigenbewegung des Schlachttieres verstanden, welche in Folge des Stimulationsreizes durch Muskelkontraktionen verursacht wird und welche als sicheres Indiz dafür gilt, dass die vorher durchgeführte Betäubung unzureichend ist.
Die Aktivbewegung unterscheidet sich hierbei signifikant von der regelmäßigen, horizontalen passiven Bewegung, welche das Schlachttier aufgrund seines Transports entlang der Rollbahn ausführt.

Der Abschnitt auf der Oberfläche des Schlachttieres, welcher in dem Bilderfassungsbereich der Bilderfassungseinheit erfasst wird, liegt hierbei in einem vorher festgelegten Bereich, in welchem die zu erwartende Aktivbewegung des Schlachttieres am größten ausfällt.

Wird durch die Auswertungseinheit eine Aktivbewegung des Schlachttieres ermittelt, so erfolgt unmittelbar eine ausgebbare Bereitstellung eines Kontrollergebnisses, welches darüber informiert, dass die Betäubung des Schlachttieres unzureichend ist.

Sowohl die Erfassung der Bildpunktdaten, als auch die Ermittlung einer eventuellen Aktivbewegung und die Bereitstellung des Kontrollergebnisses erfolgen erfindungsgemäß in Echtzeit, sodass eine unzureichende Betäubung des Schlachttieres rechtzeitig vor den anschließenden Verarbeitungsschritten, insbesondere des Entblutens oder Brühens, feststellbar ist und das Schlachttier unmittelbar nachbetäubt werden kann.

Die erfindungsgemäße Lösung beschränkt sich hierbei nicht auf das Bereitstellen des Kontrollergebnisses bei Feststellung einer Aktivbewegung. Vielmehr ist die Auswertungseinheit ebenfalls dazu in der Lage, bei Nichtfeststellung einer Aktivbewegung ein entsprechendes Kontrollergebnis auszugeben, welches dann besagt, dass die Betäubung des Schlachttieres korrekt durchgeführt wurde und wirksam ist.

Die erfindungsgemäße Vorrichtung weist insbesondere den Vorteil auf, dass, in Übereinstimmung mit geltenden und zukünftigen Tierschutzverordnungen, innerhalb des Schlachttierverarbeitungsprozesses in Echtzeit eine sichere Betäubungskontrolle des jeweiligen Schlachttieres bereitstellbar ist. Ferner besteht der Vorteil, dass die Betäubungskontrolle objektiviert durchgeführt wird und Fehler durch Unaufmerksamkeiten oder Fehlinterpretationen, wie sie bei einer visuellen Kontrolle durch Personal auftreten können, vermieden werden. Weiterhin besteht der Vorteil, dass die Kontrolle manipulationssicher aufführbar und das Kontrollergebnis dokumentierbar und speicherbar und somit archivierbar ist. Das Kontrollergebnis wird dabei vorzugsweise der Identifikation des Schlachttiers zugeordnet. Vorteilhafterweise ist die Auswertungseinheit mit einer zentralen Steuerungseinheit und einer zentralen Datenbasis des Schlachthofs verbunden. Zudem kann als weiterer Vorteil an dieser Stelle des Schlachttierverarbeitungsprozesses weitestgehend auf einen Einsatz von Personal verzichtet werden, wodurch sich insbesondere die Kosten für die Betäubungskontrolle und somit für den gesamten Schlachttierverarbeitungsprozess senken lassen.

In einer Weiterbildung der Erfindung ist die Auswertungseinheit zusätzlich mit der Stimulationseinheit verbunden und dazu in der Lage, die Abgabe des Stimulationsreizes zu steuern.
In diesem Fall ist vorzugsweise durch die Auswertungseinheit, anhand erfasster Bildpunktdaten, die korrekte Positionierung des Schlachttieres gegenüber der Stimulationseinheit ermittelbar und bei Erreichen einer festgelegten Endposition des Schlachttieres ein entsprechendes Signal zur Abgabe des Stimulationsreizes an die Stimulationseinheit übersendbar. Als besonderer Vorteil kann gemäß dieser Weiterbildung auch die Abgabe des Stimulationsreizes dem Kontrollergebnis zugeordnet sowie dokumentiert und archiviert werden.

Eine bevorzugte Weiterbildung der Erfindung sieht zudem vor, dass die 3D-Kamera als TOF-Kamera (Time Of Flight-Kamera) ausgebildet ist.

Eine derartige TOF-Kamera ergmöglicht die Ermittlung einer Distanz zwischen ihr und einem erfassten Objekt mittels Laufzeitverfahrens.
Dabei weist die TOF-Kamera insbesondere die Vorteile auf, dass diese in der Regel einen einfachen Aufbau aufweisen und somit kostengünstig bereitgestellt werden können und dass durch diese hohe Bildraten realisert werden können, indem das gesamte Objekt, vorliegend das Schlachttier oder ein relevanter Bereich des Schlachttieres, in einer Aufnahme in sehr kurzer Zeit abbildbar ist.

Ferner können die Koordinaten eines Bildpunktes als x-, y- und z-Werte, also die Raumkoordinatendaten ohne weitere Bildauswertung erhalten werden, so dass auch die Raumkoordinatendaten optisch gleichmäßiger Oberflächen unmittelbar erhalten und ausgewertet werden können, ohne dass beispielsweise ein Projektionsmuster verwandt werden müsste.

In einer besonders vorteilhaften Weiterbildung der Erfindung ist mittels der Stimulationseinheit ein berührungsloser Stimulationsreiz an das Schlachttier abgebbar.

Das Abgeben eines berührungslosen Stimulationsreizes an das Schlachttier bietet insbesondere den Vorteil, dass so eine Kontamination des Schlachttieres durch etwaige, den Stimulationsreiz übertragende, Elemente der Stimulationsvorrichtung wirkungsvoll verhindert werden kann.

In einer weiteren vorteilhaften Variante der Erfindung ist durch die Stimulationseinheit ein thermischer Stimulationsreiz an das Schlachttier abgebbar.

Die Vorteile eines thermischen Stimulationsreizes liegen hierbei vor allem in der einfachen und technisch unkomplizierten Bereitstellung und dem, in Abhängigkeit der Temperatur des Stimulationsreizes, frei wählbaren Abstand zwischen Stimulationseinheit und Schlachttier.

Der thermische Stimulationsreiz kann vorliegend beispielsweise durch einen Heißwasserstrahl bereitgestellt werden.

Bei dem thermischen Stimulationsreiz handelt es sich in einer bevorzugten Weiterbildung um einen Dampfstrahl, welcher als besonderer Vorteil sehr gezielt auf empfindliche Bereiche des Schlachttieres, beispielsweise die Nasenregion, abgegeben werden kann.

Eine vorteilhafte Ausbildung der erfindungsgemäßen Vorrichtung sieht vor, dass mittels des Kontrollergebnisses externe Einheiten steuerbar sind.

Externe Einheiten stellen in diesem Zusammenhang beispielsweise Sortiereinheiten dar, welche, bei Ermittlung einer Aktivbewegung des Schlachttieres durch die Auswertungseinheit, eine Ausschleusung des Schlachttieres aus dem laufenden Schlachttierverarbeitungsprozess und dessen Zuführung zur manuellen oder automatischen Nachbetäubung vornehmen.

Des Weiteren können externe Einheiten vorliegend Nachbetäubungseinheiten sein, welche im Anschluss an die Betäubungskontrolle in den Schlachttierverarbeitungsprozess integriert sind und welche auf Basis des Kontrollergebnisses wenn nötig eine Nachbetäubung des Schlachttieres vornehmen.

Das Kontrollergebnis wird in der hier aufgeführten Ausbildung vorzugsweise als Steuerungssignal ausgebbar bereitgestellt, welches von den externen Einheiten erfassbar und verarbeitbar ist.

Der Vorteil der vorliegenden Ausbildung der Erfindung besteht insbesondere darin, dass bei einer Ermittlung einer Aktivbewegung des Schlachttieres dieses in kürzester Zeit der Nachbetäubung zugeführt werden kann und somit ein unnötiges Leiden des Schlachttieres schnellstmöglich unterbunden werden kann. Zudem wird auf diese Weise der Schlachttierverarbeitungsprozess nur unwesentlich beeinträchtigt.

Nach einer durchgeführten Nachbetäubung wird das Schlachttier vorzugsweise erneut der Vorrichtung zur Betäubungskontrolle zugeführt, in welcher dann eine erneute Überprüfung auf die Wirksamkeit der Betäubung erfolgt.

Das durch die Auswertungseinheit bereitgestellte Kontrollergebnis ist in einer bevorzugten Weiterbildung der Erfindung einem schlachttierbezogenen Datensatz zuordenbar.

Ein solcher Datensatz ist vorzugsweise bereits in der Auswertungseinheit hinterlegt und weist insbesondere einen Schlachttieridentifikationscode, Angaben zur Art des Schlachttieres und zu dessen Maßen, sowie zu dessen Herkunftsort auf.

Die Zuordnung des Kontrollergebnisses zu dem schlachttierbezogenen Datensatz ermöglicht als besonderen Vorteil eine Archivierung der durchgeführten Betäubungskontrolle entsprechend dem jeweiligen Schlachttier und damit eine detaillierte Nachweisführung darüber, ob insbesondere aus tierschutzrechtlicher Sicht die Betäubung des Schlachttieres während der Schlachttierverarbeitung korrekt durchgeführt wurde und wirksam war.

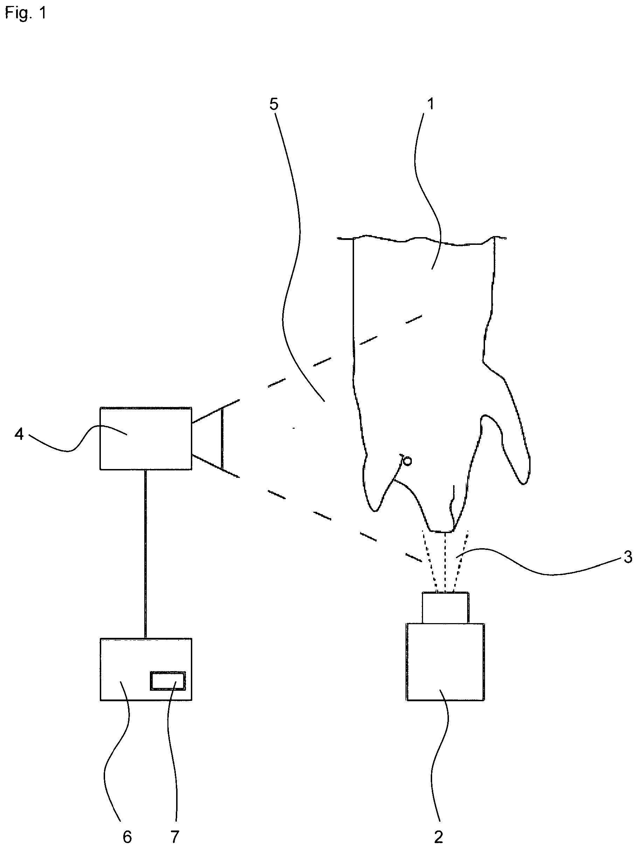

Die Erfindung wird als Ausführungsbeispiel anhand von
- Fig. 1: Prinzipdarstellung Seitenansicht
näher erläutert.

Fig. 1 zeigt die erfindungsgemäße Vorrichtung zur Betäubungskontrolle eines Schlachttieres 1 als Prinzipdarstellung in einer Seitenansicht, wobei der Übersichtlichkeit halber nur der relevante Bereich des Schlachttieres 1 dargestellt ist. Bei dem Schlachttier 1 handelt es sich vorliegend um ein Schlachtschwein.

Die erfindungsgemäße Vorrichtung ist innerhalb einer Schlachttierverarbeitung nach einer Betäubungsstation (nicht dargestellt) und insbesondere vor einer Entblutungsstation (nicht dargestellt) oder vor einer Brühstation (nicht dargestellt) vorgesehen.

Wie in Fig. 1 dargestellt, weist die Vorrichtung eine Stimulationseinheit 2 auf, mit welcher ein Stimulationsreiz 3 an das Schlachttier 1 abgebbar ist. Vorliegend ist die erfindungsgemäße Vorrichtung so innerhalb der Schlachttierverarbeitung angeordnet, dass das Schlachttier 1 kopfüberhängend, beispielsweise an einer Rollbahn (nicht dargestellt) geführt, über die Stimulationseinheit 2 hinweg bewegt wird.

Der Stimulationsreiz 3 ist im vorliegenden Ausführungsbeispiel als heißer Dampfstrahl ausgebildet, welcher insbesondere auf die empfindliche Nasenregion des Schlachttieres 1 einwirkt.
Der Bereitstellung des Stimulationsreizes 3 liegt die Überlegung zugrunde, dass das Schlachttier 1, bei einer unzureichenden Betäubung, eine körperliche Reaktion auf den Stimulationsreiz 3 ausführen würde.

Die erfindungsgemäße Vorrichtung weist ferner eine Bilderfassungseinheit 4, vorliegend als Farbwertkamera ausgebildet, mit einem Bilderfassungsbereich 5 auf.
Die Bilderfassungseinheit 4 ist hierbei gegenüber dem Schlachttier 1 so ausgerichtet, dass in dem Bilderfassungsbereich 5 ein relevanter Abschnitt der Oberfläche des Schlachttieres 1 erfassbar ist, in welchem, im Falle einer Reaktion auf den Stimulationsreiz 3 infolge mangelhafter Betäubung, eine Aktivbewegung des Schlachttieres 1, zu erwarten ist.

Die Festlegung des entsprechend relevanten Abschnittes der Oberfläche des Schlachttieres 1 wird beispielsweise anhand empirischer Untersuchungen vorgenommen. Bei einem relevanten Abschnitt kann es sich auch um das gesamte Schlachttier, beispielsweise aus seitlicher Perspektive, handeln.

Der relevante Abschnitt der Oberfläche ist in dem Bilderfassungsbereich 5 anhand von diskreten Bildpunkten erfassbar, wobei die Bildpunkte durch die Bilderfassungseinheit 4 beispielsweise mit deren Flächenkoordinaten und einem Farbwert erfassbar sind.
Die erfassten Bildpunkte werden anschließend in Echtzeit durch die Bilderfassungseinheit 4 als Bildpunktdaten übertragbar bereitgestellt.

Als weitere Komponente weist die erfindungsgemäße Vorrichtung, wie in Fig. 1 dargestellt, eine Auswertungseinheit 6 auf, welche mit der Bilderfassungseinheit 4 verbunden ist und welche die, durch die Bilderfassungseinheit 4 bereitgestellten, Bildpunktdaten erfasst.

Anhand der erfassten Bildpunktdaten ist die Auswertungseinheit 6 erfindungsgemäß dazu in der Lage, eine eventuelle Aktivbewegung des Schlachttieres 1 zu ermitteln. Hierzu werden beispielsweise in einem ersten Schritt durch die Bilderfassungseinheit 4 Bildpunkte in dem relevanten Abschnitt der Oberfläche des Schlachttieres 1 vor der Abgabe des Stimulationsreizes 3 erfasst und die entsprechenden Bildpunktdaten als Referenzbildpunktdaten von der Auswertungseinheit 6 erfasst. In einem zweiten Schritt erfolgt dann die Abgabe des Stimulationsreizes 3 an das Schlachttier 1 und die erneute, Erfassung der Bildpunkte in dem relevanten Abschnitt der Oberfläche des Schlachttieres 1. Die entsprechenden Bildpunktdaten werden anschließend von der Auswertungseinheit 6 als Vergleichsbildpunktdaten erfasst und mit den Referenzbildpunktdaten verglichen. Bei einer Aktivbewegung würde sich eine Veränderungen der Lagebeziehungen der Bildpunkte ergeben.

Stellt die Auswertungseinheit 6 während des Bildpunktdatenabgleichs fest, dass Veränderungen der Lagebeziehungen der Bildpunkte auftraten und die Veränderungen einen vorher definierten Grenzwert überschreitet, wurde durch das Schlachttier 1 eine Aktivbewegung als Reaktion auf den Stimulationsreiz 3 ausgeführt. In diesem Fall wird die Betäubung des Schlachttieres 1 durch die Auswertungseinheit 6 als nicht in Ordnung eingestuft und ein entsprechendes Kontrollergebnis, beispielsweise als rotes optisches Signal, ausgegeben. Das Schlachttier 1 wird dementsprechend, manuell oder automatisch, aus dem regulären Schlachttierverarbeitungsprozess ausgeschleust und einer Nachbetäubung zugeführt. Nach der Durchführung der Nachbetäubung erfolgt vorliegend eine nochmalige Betäubungskontrolle durch die erfindungsgemäße Vorrichtung.

Liegen dagegen im Ergebnis des Vergleichs der Vergleichsbildpunktdaten mit den Referenzbildpunktdaben keine hinreichenden Veränderungen der Lagebeziehungen der Bildpunkte vor, so wurde durch das Schlachttier 1 keine Aktivbewegung als Reaktion auf den Stimulationsreiz 3 ausgeführt. In diesem Fall wird die Betäubung des Schlachttieres 1 durch die Auswertungseinheit 6 als in Ordnung eingestuft und ein entsprechendes Kontrollergebnis, beispielsweise als grünes optisches Signal, ausgegeben. Das Schlachttier 1 wird dementsprechend, gemäß dem regulären Schlachttierverarbeitungsprozess, der Entblutung zugeführt.

Zur Ausgabe des optischen Signals ist der Auswertungseinheit 6 im vorliegenden Ausführungsbeispiel eine Anzeigeeinheit 7 zugeordnet.

In einer weiteren Ausführungsform der Erfindung erfolgt anhand des, durch die Auswertungseinheit bereitgestellten, Kontrollergebnisses eine Ansteuerung externer Einheiten (nicht dargestellt). Bei derartigen externen Einheiten handelt es sich vorliegend insbesondere um Sortiereinheiten, welche das Schlachttier 1, im Falle einer unzureichenden Betäubung, vollautomatisch aus dem Schlachttierverarbeitungsprozess ausschleusen und der Nachbetäubung zuführen.

In dieser Ausführungsform der Erfindung wird das Kontrollergebnis durch die Auswertungseinheit 6 zusätzlich als Steuerungssignal ausgegeben, welches wiederrum von den externen Einheiten erfassbar und verarbeitbar ist.

Die einzelnen durch die erfindungsgemäße Vorrichtung ausführbaren Schritte Stimulationsreizabgabe, Bilderfassung, Bewegungsermittlung und Kontrollergebnisausgabe erfolgen im Ausführungsbeispiel in Echtzeit, sodass zum einen der laufende Schlachttierverarbeitungsprozess nicht unnötig gestört wird und zum anderen eine unzureichende Betäubung des Schlachttieres 1 schnellstmöglich erkannt und eine entsprechende Nachbetäubung vorgenommen werden kann.

### Verwendete Bezugszeichen

- 1: Schlachttier
- 2: Stimulationseinheit
- 3: Stimulationsreiz
- 4: Bilderfassungseinheit
- 5: Bilderfassungsbereich
- 6: Auswertungseinheit
- 7: Anzeigeeinheit

## Patentansprüche

1. Vorrichtung zur Betäubungskontrolle eines Schlachttieres (1), aufweisend eine Stimulationseinheit (2), mit welcher ein Stimulationsreiz (3) an das Schlachttier (1) abgebbar ist, und aufweisend eine Bilderfassungseinheit (4) mit einem Bilderfassungsbereich (5), wobei die Bilderfassungseinheit (4) als 3D-Kamera ausgebildet ist und wobei in dem Bilderfassungsbereich ein Abschnitt einer Oberfläche des Schlachttieres (1) optisch erfassbar ist und in welchem Bildpunkte in dem Abschnitt der Oberfläche erfassbar und als Bildpunktdaten übertragbar bereitstellbar sind, und aufweisend eine Auswertungseinheit (6), welche mit der Bilderfassungseinheit (5) verbunden ist und welche die durch die Bilderfassungseinheit (5) bereitgestellten Bildpunktdaten erfasst und mit welcher aus den erfassten Bildpunktdaten eine Aktivbewegung des Schlachttieres (1) ermittelbar ist und mit welcher, bei einer ermittelten Aktivbewegung des Schlachttieres (1), ein Kontrollergebnis ausgebbar bereitstellbar ist.

2. Vorrichtung nach Anspruch 1,
wobei die 3D-Kamera als TOF-Kamera ausgebildet ist.

3. Vorrichtung nach Anspruch 1,
wobei mittels der Stimulationseinheit (2) ein berührungsloser Stimulationsreiz abgebbar ist.

4. Vorrichtung nach Anspruch 2,
wobei durch die Stimulationseinheit (2) ein thermischer Stimulationsreiz abgebbar ist.

5. Vorrichtung nach Anspruch 3,
wobei es sich bei dem thermischen Stimulationsreiz um einen Dampfstrahl handelt.

6. Vorrichtung nach einem der vorhergehenden Ansprüche,
wobei mittels des bereitgestellten Kontrollergebnisses externe Einheiten steuerbar sind.

7. Vorrichtung nach einem der vorhergehenden Ansprüche, wobei das bereitgestellte Kontrollergebnis einem schlachttierbezogenen Datensatz zuordenbar ist.

## Claims

1. A device for the anaesthesia control of a slaughter animal (1) comprising a stimulation unit (2) by means of which a stimulation impulse (3) can be delivered to the slaughter animal (1) and comprising an image acquisition unit (4) with an image acquisition range (5), wherein the image acquisition unit (4) is designed as a 3D camera and wherein in the image acquisition range a portion of a surface of the slaughter animal (1) can be optically recorded and in which pixels in the portion of the surface can be recorded and made available as transferable pixel data, and comprising an analysis unit (6), which is connected to the image acquisition unit (5) and which registers the pixel data provided by the image acquisition unit (5) and by means of which an active movement of the slaughter animal (1) can be determined from the pixel data recorded and by menas of which a control result can be provided as an output if an active movement of the slaughter animal (1) has been determined.

2. The device according to claim 1,
wherein the 3D camera is designed as a TOF camera.

3. The device according to claim 1,
wherein a contactless stimulation impulse can be delivered by the stimulation unit (2).

4. The device according to claim 2,
wherein a thermal stimulation impulse can be delivered by the stimulation unit (2).

5. The device according to claim 3,
wherein the thermal stimulation impulse is a steam jet.

6. The device according to one of the previous claims,
wherein external units can be controlled by means of the control result provided.

7. The device according to one of the previous claims,
wherein the control result provided can be assigned to a data record related to slaughter-animal.

## Revendications

1. Dispositif de contrôle de l'anesthésie d'un animal de boucherie (1) qui comporte une unité de stimulation (2) à l'aide de laquelle un stimulus (3) peut être appliqué à l'animal de boucherie (1), et une unité d'acquisition d'images (4) comportant une zone d'acquisition d'images (5), ladite unité d'acquisition d'images (4) étant conçue sous forme de caméra 3D et une zone d'une surface de l'animal de boucherie (1) pouvant être acquise optiquement dans la zone d'acquisition d'images, dans laquelle des pixels peuvent être collectés dans la zone de la surface et peuvent être transmis et fournis en tant que données de pixels, ainsi qu'une unité d'évaluation (6) qui est connectée à l'unité d'acquisition d'images (5), et qui collecte les données de pixels fournies par l'unité d'acquisition d'images (5), ladite unité d'évaluation permettant de déterminer un mouvement actif de l'animal de boucherie (1) à partir des données de pixels collectées et de transmettre et de fournir un résultat de contrôle en cas de mouvement actif déterminé de l'animal de boucherie (1).

2. Dispositif suivant la revendication 1
dont la caméra 3D est conçue sous forme de caméra TOF.

3. Dispositif suivant la revendication 1
dont l'unité de stimulation (2) peut appliquer un stimulus sans contact.

4. Dispositif suivant la revendication 2
dont l'unité de stimulation (2) peut appliquer un stimulus thermique.

5. Dispositif suivant la revendication 3,
dont le stimulus thermique est un jet de vapeur.

6. Dispositif suivant une des revendications précédentes
dont des unités externes peuvent être contrôlés au moyen du résultat de contrôle fourni.

7. Dispositif suivant une des revendications précédentes dont le résultat de contrôle fourni peut être attribué à un ensemble de données concernant l'animal de boucherie.
